# FASCICULE DE BREVET EUROPEEN

(11) **EP 1 109 462 B1**
(45) Date de publication et mention de la délivrance du brevet: **12.11.2003**
(21) Numéro de dépôt: 99940241.5
(22) Date de dépôt: 27.08.1999
(51) Int. Cl.: A23L 1/312, A23J 1/10

(54) **PROCEDE D'OBTENTION DE PRODUITS BIOLOGIQUES AVIAIRES**
VERFAHREN ZUR HERSTELLUNG VON BIOLOGISCHEN GEFLÜGELPRODUKTEN
METHOD FOR OBTAINING AVIAN BIOLOGICAL PRODUCTS

(30) Priorité: 31.08.1998 FR 9810868
(43) Date de publication de la demande: 27.06.2001
(73) Titulaire: Diana Ingredients, 56250 Saint Nolff (FR)
(72) Inventeur: MOLLARD, Laurent, F-56000 Vannes (FR); MONTILLET, Agnès, F-44600 Saint-Nazaire (FR); HORRIERE, Cécile, F-56000 Vannes (FR); LEGRAND, Jack, F-44600 Saint-Nazaire (FR); NGUYEN, Tan, Hung, F-56890 Saint-Avé (FR)
(74) Mandataire: Ahner, Francis
(86) Numéro de dépôt international: FR9902052
(87) Numéro de publication internationale: WO00011969

(56) Documents cités:
- US-A- 3 900 579
- US-A- 5 384 149
- US-A- 5 637 321

## Description

La présente invention concerne un procédé de séparation et d'extraction de produits biologiques d'origine aviaire. Le procédé permet l'obtention de cartilages aviaires et des principes actifs qu'on peut extraire des cartilages ainsi obtenus.

### Etat de l'art

Les cartilages sont des tissus complexes qu'on trouve dans de nombreux organes des humains et des animaux.

Ainsi, on peut prélever des cartilages au niveau des cloisons nasales, du larynx, de la trachée artère, des bronches, des surfaces articulaires, des cartilages de conjugaison des os longs, de la pointe xyphoïde du sternum, etc...

Chez les poissons chondrichthyens (requins, roussettes, raies, etc...), l'ensemble du squelette est cartilagineux.

Les cartilages sont constitués de nombreuses molécules utilisées comme principes actifs en alimentation diététique humaine et animale, en pharmacie humaine et vétérinaire ou en cosmétologie. Parmi les molécules les plus connues on peut citer: les collagènes, les hexosamines et les glycosaminoglycanes (chondroïtine sulfate, kératane sulfate, l'acide hyaluronique).

La majorité de ces molécules sont jusqu'à maintenant extraites de cartilages de bovins. Cependant, depuis l'apparition de l'encéphalite spongiforme bovine (ESB), les industries alimentaire, pharmaceutique et cosmétique s'inquiètent d'une éventuelle contamination de ces extraits par les prions responsables de l'ESB et difficiles à détecter.

L'emploi de squelettes de poissons chondrichtyens peut être une solution de substitution aux produits d'origine bovine. Mais les ressources marnes ont des limites à la fois quantitatives, économiques et environnementales.

Il est donc utile de trouver une autre source de cartilages provenant d'animaux abondants et reconnus comme indemnes de maladies à prions. Les volailles domestiques (poulets, dindons, canards, pintades, cailles, pigeons) répondent à ces critères de sécurité sanitaire.

Sur le squelette des oiseaux, les cartilages utilisables se trouvent principalement sur la pointe du sternum (bréchet), sur les surfaces articulaires, et au niveau des cartilages de conjugaison des os longs.

Cependant, ces cartilages ne représentent qu'une part très faible du squelette des oiseaux et nous ne connaissons pas de procédé capable de les séparer et les extraire efficacement en vue d'une production industrielle. Ainsi, par exemple, le brevet US 5 637 321 décrit un prélèvement manuel, après dissection au couteau, de cartilages de poulets utilisables pour obtenir du collagène de type II utile dans le traitement de l'arthrite. Un tel procédé manuel ne permet pas une production industrielle de masse.

### L'invention

Nous avons inventé un procédé mécanisé qui permet la séparation et l'extraction des cartilages des squelettes des oiseaux domestiques.

Le procédé consiste à broyer des squelettes d'oiseaux domestiques et à soumettre le broyat à un flux de liquide qui circule dans un récipient de séparation. De façon avantageuse, ledit flux de liquide présente une composante verticale ascendante.

Il s'est avéré avantageux de broyer les squelettes des oiseaux afin d'obtenir des particules de tailles inférieures à environ un centimètre.

Le liquide de séparation utilisable peut être simplement de l'eau ou de la saumure formée d'eau et d'un sel comestible. Dans ce dernier cas, on peut utiliser avantageusement du sel de cuisine (NaCl) pour fabriquer une saumure contenant moins de 32,5% de sel.

La forme du récipient de séparation ainsi que la hauteur du liquide de séparation ont peu d'importance. L'essentiel est que le liquide de séparation puisse se déplacer librement. Les débits du liquide de séparation sont à régler en fonction de la structure des squelettes qui peut varier avec les espèces animales et l'âge des oiseaux. La taille du récipient de séparation doit varier en fonction des quantités de produits à traiter.

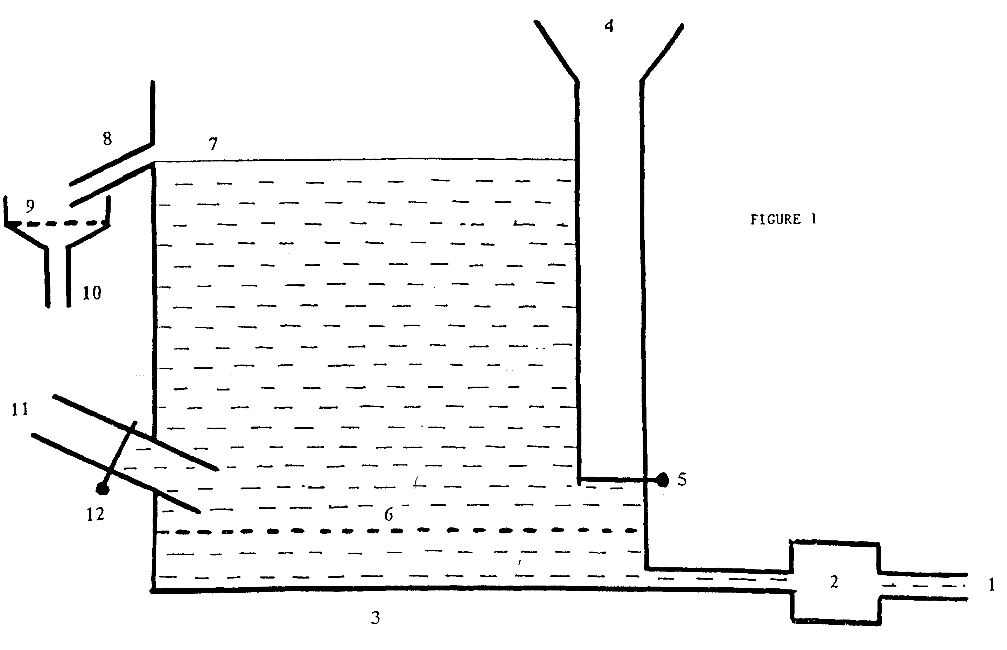

Comme exemples non exhaustifs et non limitatifs, la figure 1 ainsi que les essais suivants vont permettre de mieux comprendre l'invention.

### Figure 1

Un cycle de séparation et d'extraction des cartilages se fait selon le principe de la figure 1 (la figure ne donne pas d'échelle ni de dimension du dispositif).

La conduite 1 amène de l'eau ou de la saumure dans le récipient de séparation 3 grâce à la pompe 2 qui en règle le débit.

On introduit un broyat de squelettes de volailles dans la conduite 4, on ouvre la vanne 5, on fait descendre le broyat jusque dans le bas du récipient 3 et au dessus de la grille 6.

Les tissus osseux restent sur la grille 6 dont les mailles sont inférieures à la taille des particules de squelettes broyés. Les tissus cartilagineux sont entraînés par le liquide de séparation à sa surface 7 et sont évacués par le trop plein 8. Ils sont ensuite recueillis dans le tamis 9. L'excès de liquide est renvoyé par la conduite 10 vers la réserve de liquide qui se situe en amont de la conduite 1. On évacue les tissus osseux par la conduite 11 après avoir ouvert la vanne 12.

Il est bien sûr facile d'automatiser l'introduction des squelettes broyés dans le récipient de séparation, l'évacuation des cartilages et des tissus osseux par tout moyen connu.

### Essai 1

Pour cet essai et l'essai suivant, le système expérimental de séparation et d'extraction des cartilages, répondant à la figure 1, comprend un récipient de séparation en plexiglass translucide de 15 litres de capacité.

La pompe a un débit variable et réglable de 0 à 3 500 litres par heure.

Pour les besoins des expériences, on évacue les tissus osseux à la fin de chaque expérience en les aspirant avec un tuyau souple relié à une pompe aspirante.

Dans ce premier essai, on prélève des squelettes de dindons qui sont des sous-produits d'un abattoir qui pratique la «découpe» de ces oiseaux.

Ensuite on les broie dans un hachoir couramment utilisé en charcuterie qu'on appelle aussi un «cutter», jusqu'à obtenir des particules de moins d'un centimètre.

Le liquide de séparation est de la saumure contenant 30% de sel de cuisine.

La pompe est réglée de telle façon que le débit de la saumure dans le récipient de séparation est de 1 500 litres par heure.

Après avoir introduit dans le système de séparation un poids total d'un kilogramme de squelettes broyés, on a récupéré dans le tamis 32 grammes de cartilages à la fin de l'expérience.

### Essai 2.

On récupère 10 kilogrammes de squelettes de poulets qui ont été grossièrement broyés dans un centre de découpes avicoles.

On les broie de nouveau au hachoir afin de réduire la taille des particules à moins d'un centimètre.

Pour cette expérience, on utilise de l'eau du robinet comme liquide de séparation.

Le débit de la pompe est réglé de façon que l'eau traverse le récipient de séparation à raison de 3 000 litres par heure.

Après l'expérience qui a porté sur les 10 kilogrammes de squelettes broyés de poulets, on a pu séparer et extraire 550 grammes de cartilages.

### Essai 3

A partir des cartilages obtenus dans l'essai 2, on procède à l'évaluation des principes actifs utilisables en alimentation diététique humaine et animale, en pharmacie humaine et vétérinaire ou en cosmétologie.

Les collagènes ont été dosés selon la méthode pratiquée par le laboratoire Laréal, 56250 Saint Nolff, France, et accréditée par le Comité Français d'Accréditation, plus connu sous le nom de COFRAC (Référence COFRAC: CC 70; Référence laboratoire: AN 85; Numéro d'accréditation: 1-285).

Les hexosamines ont été dosées selon la méthode décrite dans les «Techniques d'analyse et de contrôle dans les industries agro-alimentaires», 1981, Volume 4, pages 95-97, éditées par Technique et Documentation, Lavoisier, APRIA.

Les glycosaminoglycanes, exprimés sous forme de chondroïtine sulfate, ont été extraits selon la méthode de L. Rodén *et al*. (*In* «Methods in Enzymology. Vol. XXVIII, Complex Carbohydrates, Part B», Edited by V. Ginsburg, Academic Press, 1972, pages 73-140), et dosés selon la méthode décrite dans Pharmeuropa, 1997, Vol. 9, N°12, pages 193-196.

Les résultats obtenus ont été les suivants, exprimés en pourcentage de poids de cartilages humides :
- collagènes : 8,80 %.
- hexosamines : 0,99 %.
- glycosaminoglycanes : 2,32 %.

## Revendications

1. Procédé de séparation et d'extraction de cartilages à partir de squelettes de volailles, en vue de la fabrication des produits biologiques contenus dans lesdits cartilages **caractérisé en ce que** les squelettes de volailles sont broyés jusqu'à obtention d'une dimension moyenne de particules inférieure à environ 1 centimètre, et introduits dans un récipient de séparation où ils sont séparés et extraits par un flux de liquide comestible circulant dans ledit récipient de séparation.

2. Procédé selon la revendication 1, **caractérisé en ce que** le flux de liquide comestible circulant dans ledit récipient de séparation présente une composante verticale ascendante.

3. Procédé selon l'une des revendications 1 ou 2, **caractérisé en ce que** le liquide de séparation est de l'eau ou une saumure comestible.

## Patentansprüche

1. Verfahren zur Abtrennung und Extraktion von Knorpeln ausgehend von Geflügelskeletten im Hinblick auf die Herstellung von biologischen Produkten, die in den Knorpeln enthalten sind, **dadurch gekennzeichnet, daß** die Geflügelskelette bis zum Erhalt einer mittleren Teilchenabmessung von weniger als etwa 1 Zentimeter gemahlen werden und in einen Abtrennungsbehälter eingeführt werden, wo sie durch einen Strom von genießbarer Flüssigkeit, die in dem Abtrennungsbehälter zirkuliert, getrennt und extrahiert werden.

2. Verfahren nach Anspruch 1, **dadurch gekennzeichnet, daß** der Strom von genießbarer Flüssigkeit, der in dem Abtrennungsbehälter zirkuliert, eine vertikale aufsteigende Komponente aufweist.

3. Verfahren nach einem der Ansprüche 1 oder 2 , **dadurch gekennzeichnet, daß** die Abtrennungsflüssigkeit Wasser oder eine genießbare Salzlake ist.

## Claims

1. Method for separating and extracting cartilages from poultry skeletons with a view to manufacturing the biological products contained in the said cartilages, **characterized in that** the poultry skeletons are ground until a mean particle size of less than about 1 centimetre is obtained and introduced into a separating vessel where they are separated and extracted by a flow of edible liquid circulating in a separating vessel.

2. Process according to Claim 1, **characterized in that** the flow of edible liquid circulating in the said separating vessel has an ascending vertical component.

3. Process according to either of claims 1 and 2, **characterized in that** the separating liquid is water or an edible brine.
